(19)

Europäisches
Patentamt
European
Patent Office
Office européen
des brevets

(11) **EP 2 508 883 A1**

(12) **EUROPEAN PATENT APPLICATION**

(43) Date of publication:
**10.10.2012 Bulletin 2012/41**

(51) Int Cl.:
**$G01N\ 33/18$** *(2006.01)*

(21) Application number: **11161097.8**

(22) Date of filing: **05.04.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **SIEMENS AKTIENGESELLSCHAFT
80333 München (DE)**

(72) Inventor: **Sichel, Cosima
89077, Ulm (DE)**

(54) **Method and apparatus for determining a chlorate concentration in water**

(57) The invention relates to a method and an apparatus for determining a chlorate concentration in a process of treating water with chlorine dioxide as a disinfectant, wherein the method comprises determining a concentration of the disinfectant in the water, determining a chlorite concentration in the water, providing a function to determine the chlorate concentration depending on the concentration of the disinfectant and the chlorite concentration based on known chemical relations for the generation of chlorate and chlorite in the process of treating water with the disinfectant, and applying the function to determine the chlorate concentration based on the concentration of disinfectant and the chlorite concentration.

## FIG 1

~ 10

EP 2 508 883 A1

## Description

The present invention relates to a method and an apparatus for determining a chlorate concentration in a process of treating water with chlorine dioxide as a disinfectant.

In order to minimize the risk of infectious diseases, it is customary to disinfect water meant for human use. In special applications such as installations for supplying non-stationary drinking water, or the treatment of water in swimming pools, disinfection is a basic necessity. Chlorine and its species, such as, sodium hypochlorite, calcium hypochlorite or chlorine gas are the most commonly used disinfectants for disinfecting water because they are economical and have good germicidal ability. However, the aforementioned disinfectants react with organic material in the water and produce various Disinfection By Products (DBPs). For example, the disinfection by-products include chloramines and trihalomethanes (THMs). These by-products are restricted in many places, and especially trihalomethanes, as the same is suspected to be a carcinogen.

Alternatively, chlorine dioxide has been proposed for disinfecting water as the same is effective in the inactivation of the pathogenic organisms and unlike chlorine and its species mentioned above does not produce trihalomethanes. However, chlorine dioxide reacts in water to produce inorganic DBPs, such as, chlorite and chlorate. These DBPs are restricted in many places, as exposure to the same at low levels may lead to hemolytic anemia, while higher levels may result in an increase in methemoglobin. Thus, standards for the concentration of chlorite and chlorate in water have been established in many countries. For example, World Health Organization (WHO) recommends an upper limit of concentration for chlorate of 1 mg/l in drinking water.

It is an object of the embodiments of the invention to provide a method and an apparatus for determining a chlorate concentration in water.

The above object is achieved by a method for determining a chlorate concentration in a process of treating water with chlorine dioxide as a disinfectant according to claim 1 and an apparatus according to claim 14.

The concentration of chlorate is computed using the concentration of the disinfectant and the concentration of chlorite in the water. Use of concentration of chlorite for the computation of the concentration of chlorate at stable water conditions enables in computing the concentration of chlorate without measuring the organic carbon content of the water directly. This enables in continuous monitoring of the concentration of chlorate. Additionally, as the organic carbon content is not required to be measured, the determination of the chlorate concentration is relatively less expensive.

According to an embodiment, the dependency of the function on the concentration of the disinfectant and/or the chlorite concentration is adjusted based on at least one calibration measurement. This achieves in computing the concentration of chlorate in the water with increased accuracy.

According to another embodiment, the concentration of the disinfectant is determined by measuring the concentration of the disinfectant in the water. This enables in deploying a sensor to measure the concentration of the disinfectant from the water directly.

According to yet another embodiment, the function is based on the chemical relations in the form of a chemical relationship for the chlorite concentration depending on the concentration of the disinfectant and in form of a chemical relationship for the chlorate concentration depending on the concentration of the disinfectant. The formation of chlorite and chlorate in water is dependent on the concentration of the disinfectant. The function being based on these chemical relations enables in determining the chlorate concentration using the concentration of the disinfectant and the chlorite concentration in the water.

According to yet another embodiment, the chemical relationships for the chlorite concentration and the chlorate concentration depend on the organic carbon content in the water and the function is established by eliminating the organic carbon content in the chemical relationships. This achieves is determining the chlorate concentration in the water without measuring the organic carbon content of the water.

According to yet another embodiment, wherein the chlorite concentration is determined by a measurement of chlorite concentration in the water. This enables in deploying a sensor to measure the chlorite concentration from the water directly.

According to yet another embodiment, the chlorite concentration is determined based on a calibration measurement of the chlorite concentration in the water, wherein the chlorite concentration is assumed as constant in the subsequent application of the function. This eliminates the requirement of using a sensor to measure the chlorite concentration.

According to yet another embodiment, the chlorite concentration is determined based on a difference of the concentration of the disinfectant which is measured at two different time instances. This eliminates the requirement of using a sensor to measure the chlorite concentration.

According to yet another embodiment, the function includes at least one additional parameter which influences the water treatment process. Including one or more additional parameters influencing the water treatment process in the function enables in determining the chlorate concentration in the water with increased accuracy.

According to yet another embodiment, the at least one additional parameter comprises at least one of temperature, pH value and reaction time of the disinfectant with the water.

According to yet another embodiment, the method may further comprise a step of measuring at least one value for the at least one additional parameter.

According to yet another embodiment, the steps of determining the concentration of the disinfectant, determining the chlorite concentration, and applying the function to determine the chlorate concentration, are repeated continuously. This achieves in determining the chlorate concentration in the water in a continuous manner.

According to yet another embodiment, the step of measuring the at least one value for the at least one additional parameter is repeated continuously. The additional parameters can be measured continuously for determining the chlorate concentration in a continuous manner.

Another embodiment includes, an apparatus for determining a chlorate concentration in a process of treating water with chlorine dioxide as a disinfectant, comprising means for determining a concentration of the disinfectant in the water, means for determining a chlorite concentration in water, and a processor configured to determine the chlorate concentration depending on the concentration of the disinfectant and the chlorite concentration by applying a function based on known chemical relations of formation of chlorate and chlorite in the process of treating water with the disinfectant.

Embodiments of the present invention are further described hereinafter with reference to illustrated embodiments shown in the accompanying drawings, in which:

FIG 1     is a flow diagram illustrating an exemplary method of determining a concentration of chlorate in water treated with chlorine dioxide as a disinfectant according to an embodiment herein,

FIG 2     illustrates an exemplary block diagram of an apparatus 35 for determining a concentration of chlorate in water being treated with chlorine dioxide as a disinfectant according to a first embodiment herein,

FIG 3     illustrates an exemplary block diagram of an apparatus 35 for measuring a concentration of chlorate in water according to a second embodiment, and

FIG 4     illustrates an exemplary block diagram of a system for disinfecting water with a chlorine dioxide based disinfectant according to an embodiment herein.

Various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

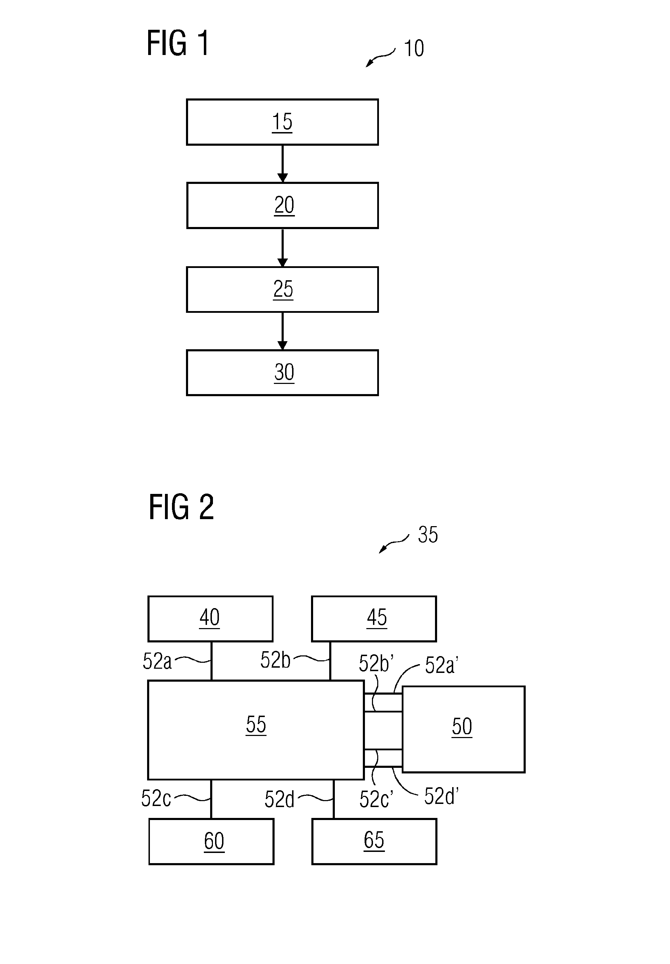

FIG 1 is a flow diagram illustrating an exemplary method 10 of determining a concentration of chlorate in water treated with chlorine dioxide as a disinfectant according to an embodiment herein. Chlorine dioxide is decomposed to chlorite and chlorate in such treatment process. At block 15 of the method 20, a concentration of the disinfectant in the water being treated is determined. For example, in an aspect, the concentration of the disinfectant in the water can be determined by measuring the concentration of the disinfectant in the water. In another aspect, the concentration of the disinfectant in the water can be determined by measuring the amount of disinfectant being added into the water and then determining the disinfectant remaining in the water after a time $t_d$ of addition of the disinfectant. In an aspect, the determination of the disinfectant remaining in the water may include considering the parameters which influence the decomposition of the disinfectant in the water, such as a pH value of the water, a temperature of the water, a reaction time of the disinfectant with the water, and the like.

Next at block 20, a chlorite concentration in the water is determined. In an aspect, the chlorite concentration can be determined by measuring the chlorite concentration in the water using a sensor. In another aspect, the chlorite concentration can be determined based on a calibration measurement of the chlorite concentration in the water. Thus, in case the chlorite concentration is determined based on the calibration measurement, the determined chlorite concentration can be assumed as constant for the subsequent computation of the chlorate concentration in the water until the next calibration measurement. According to another aspect, the chlorite concentration can be determined based on a difference of the concentration of the disinfectant at two different time instances. The difference of the concentration of the disinfectant at two different time instances provides an amount of consumption of the disinfectant in the water. Consumption of the disinfectant in the water means the amount of disinfectant which has been decomposed in the water over the time period between the two time instances. Generally, the concentration of chlorite in the water increases with an increase in the consumption of the disinfectant. Thus, based on this principle, the chlorite concentration can be determined based on the difference in the concentration of the disinfectant at two different time instances.

Next at block 25, according to an aspect, a function is provided to determine the chlorate concentration in the water depending on the concentration of the disinfectant in the water and the chlorite concentration in the water. According to an aspect, the function is derived using known chemical relations for the generation of chlorite and chlorate in the process of treating the water with the disinfectant. The known chemical relations can be observed from the publication of Lee et., al, Formation of chlorite and chlorate from chlorine dioxide with Han river water, Korean J. Chem. Eng., (Vol. 21, No. 3). The publication discloses following models obtained by regression analysis for the formation of chlorite and chlorate in the reaction of a sample with chlorine dioxide:

$$\text{Chlorite (mg/L)} = 10^{-2.20}[ClO_2]^{0.45}[pH]^{0.90}[temp]^{0.27}[TOC]^{1.04}[time]^{0.20} \quad (1)$$

$$\text{Chlorate (mg/L)} = 10^{-2.61}[ClO_2]^{1.27}[pH]^{-0.5}[temp]^{1.28}[TOC]^{0.31}[time]^{0.12} \quad (2)$$

where, $ClO_2$ is the concentration of chlorine dioxide in the sample, pH is the pH value of the sample, temp is temperature of the sample in °C, TOC is the total organic carbon content of the sample in mgC/L and time is the reaction time of chlorine dioxide with the sample in s. From equations (1) and (2) it can be seen that the concentration of chlorite and chlorate in the sample depend on the concentration of chlorine dioxide, pH value of the sample, temperature of the sample, TOC of the sample, and reaction time of chlorine dioxide with the sample.

Referring still to block 25, advantageously, the dependency of the function on the concentration of the disinfectant and/or the chlorite concentration can be adjusted based on a calibration measurement. In an aspect, the calibration measurement can be performed using the water for which the chlorate concentration is to be determined. For example, the calibration measurement can include determining a calibration constant and the function can include the calibration constant. According to an embodiment, the function can be expressed mathematically as per the following equation:

$$\text{Chlorate (mg/L)} = a(C^{x_1})(X^{x_2}) \quad (3)$$

Where, a is the calibration constant, C is the concentration of the disinfectant in the water in mg/L, $X$ is the chlorite concentration in the water in mg/L and $x_1$, $x_2$ are exponential constants. The exponential constants $x_1$ and $x_2$ can be determined using experimental methods and may vary for different apparatus for measuring the chlorate concentration in water and different type of sensors. The experimental methods can also include regression analysis for deriving the exponential constants. Advantageously, the exponential constants $x_1$, $x_2$ can be determined prior to the determination of the chlorate concentration in the water and can be used in equation (1) for determining the chlorate concentration.

According to an embodiment herein, the calibration constant a can be determined using the water for which the chlorate concentration is to be determined. The calibration constant a is determined prior to the process of determining the chlorate concentration in the water and can be repeated periodically. In certain aspects, the repetition period can be predetermined. For example, for determining the calibration constant a, the chlorate concentration in the water can be measured using known techniques, such as, ion chromatography, titration method or photometric method and the like. The concentration of the disinfectant and the chlorite concentration in the water can be determined as per the embodiments described herein.

Referring still to block 25, according to an aspect, to achieve increased accuracy in the determination of the chlorate concentration in the process of treating water with the disinfectant, the function for determining the chlorate concentration in the water may include one or more additional parameters that influence the water treatment process. From equations (1) and (2) it can be seen that the formation of chlorate and chlorite is also influenced by additional parameters, such as temperature, pH value and reaction time of the disinfectant with the sample. Thus, the more the number of additional parameters being included in the function, the determination of the chlorate concentration can be more accurate. According to an aspect, the additional parameters to be included in the function can be measured. For example, the temperature and the pH value of the water can be measured from the water. The reaction time of the disinfectant with the water t can be measured as the time elapsed after the addition of the disinfectant to the water. The function including the additional parameters can be expressed mathematically as per the following equation:

$$\text{Chlorate (mg/L)} = a(C^{x_1})(X^{x_2})(pH^{x_3})(T^{x_4})(t^{x_5}) \quad (4)$$

where, $C$ is the concentration of the disinfectant in the water in mg/L, $X$ is the chlorite concentration in mg/L, $pH$ is the pH value of the water, $T$ is the temperature of the water in °C, $t$ is the reaction time of the disinfectant with water in s and $x_1$, $x_2$, $x_3$, $x_4$, $x_5$ are the respective exponential constants. The exponential constants can be determined as explained in block 20. The additional parameters can be measured online in a very inexpensive manner. This enables in more accurate chlorate concentration prediction with inexpensive tools. Equation (4) has been depicted as including the additional parameters, pH value of the water, temperature of the water and the reaction time of the disinfectant with the water. However, one or more additional parameters may be included in the function. However, the more the additional parameters are included in the function for determining the chlorate concentration, higher accuracy can be achieved.

According to an aspect, the function can further include an amount of consumption of the disinfectant in the water. Generally, the consumption of the disinfectant in the water increases with the increase in the organic carbon content in the water. The amount of consumption of the disinfectant provides the amount of the disinfectant that has reacted with the organic matter present in the water over a reaction time period. Generally, the consumption of the disinfectant increases if the organic carbon content of the water is high and decreases if the organic carbon content is low. The organic carbon content of the water is one of the reaction parameters that influence the formation of chlorate. The function including the amount of consumption of the disinfectant is expressed mathematically as per the following equation:

$$\text{Chlorate (mg/L)} = a(C^{x_1})(X^{x_2})(\Delta C^{x6}) \tag{5}$$

where, $C$ is the concentration of the disinfectant in the water in mg/L, $X$ is the chlorite concentration in the water in mg/L and $\Delta C$ is the amount of consumption of the disinfectant in the water in mg/L and $x_6$ is the respective exponential constant. The exponential constant $x_6$ can be determined similar to the other exponential constant as explained in block 20. Thus, using the amount of consumption of the disinfectant in the water for determining the chlorate concentration as per equation (5) enables in achieving increased accuracy in the computation of the concentration of chlorate in the water without measuring the organic carbon content in the water directly. Equation (5) expresses the function being dependent on the concentration of the disinfectant, the chlorite concentration and the amount of consumption of the disinfectant. However, the function including the amount of consumption can further include one or more of the additional parameters influencing the formation of chlorite and chlorate as expressed by the following equation:

$$\text{Chlorate (mg/L)} = a(C^{x_1})(X^{x_2})(pH^{x_3})(T^{x_4})(t^{x_5})(\Delta C^{x_6}) \tag{6}$$

Where, $C$ is the concentration of the disinfectant in the water in mg/L, $X$ is the chlorite concentration in mg/L, pH is the pH value of the water, $T$ is the temperature of the water in °C, $t$ is the reaction time of the disinfectant with water in s, $\Delta C$ is the amount of consumption of the disinfectant in the water in mg/L and $x_1$, $x_2$, $x_3$, $x_4$, $x_5$, $x_6$ are the respective exponential constants.

Next, at block 30, the chlorate concentration is determined by applying the function based on the concentration of the disinfectant and the chlorate concentration in the water. Using the concentration of chlorite together with the concentration of the disinfectant in the water to compute the concentration of chlorate enables in taking into the consideration the organic carbon content of the water indirectly. Thus, the chlorate concentration in the water can be determined in a continuous manner as organic carbon content in the water is not required to be measured directly. For example, if the concentration of the disinfectant and the chlorite concentration are determined by measurements from the water, the chlorate concentration in the water can be determined at each measurement cycle. Additionally as the organic carbon content is not required to be measured, the concentration of chlorate can be measured at a faster rate and less expensively. Moreover, if the function includes one or more of the additional parameters, the additional parameters can also be measured repeatedly for determining the chlorate concentration in a continuous manner.

FIG 2 illustrates an exemplary block diagram of an apparatus 35 for determining a concentration of chlorate in water being treated with chlorine dioxide as a disinfectant according to a first embodiment herein. The apparatus 35 comprises a first sensor 40 and a second sensor 45 operably coupled to a processor 50. In the shown example of FIG 1, the sensor 40 is adapted to measure the concentration of the disinfectant in the water and the sensor 45 is adapted to measure the chlorate concentration in the water.

The first sensor 40 is configured to output a first signal 52a responsive to the measured concentration of the disinfectant and the second sensor 45 is configured to output a second signal 52b responsive to the measured chlorite concentration in the water. The first signal 52a and the second signal 52b outputted by the sensors 40, 45 are converted from analog to digital by an analog to digital converter 55 and provided to the processor 50, depicted as 52a' and 52b' respectively. The processor 50 is configured to receive the digitized first signal 52a' and the second signal 52b' and extract the value indicated by the digitized first signal 52a' as an indication of the concentration of the disinfectant in the water and the value indicated by the digitized second signal 52b' as an indication of the chlorite concentration in the water. According to an aspect, the processor 50 is configured to determine the chlorate concentration in the water using the function as expressed in equation (3). The function can be stored into a memory internal or external to the processor 50 and the processor 50 can extract the function for determining the chlorate concentration in the water. As discussed above, the exponential constants $x_1$ and $x_2$ can be determined prior to the apparatus 10 being installed for determining the chlorate concentration in the water and can be stored in the memory.

Referring still to FIG 2, a calibration measurement is performed to determine the calibration constant a when the apparatus 35 is being installed at the site of the water before initiating the process of determining the chlorate concentration in the water. For example, according to an aspect, the chlorate concentration in the water can be determined manually using the techniques described above and provided to the processor 50. For example, a user can determine the chlorate concentration and provide the same to the processor 50 as an input via an input user interface operably coupled to the processor 50. The concentration of the disinfectant and the chlorite concentration in the water can be measured by the first sensor 40 and the second sensor 45 respectively and provided to the processor 50. The calibration constant a can then be computed by the processor 50 using the chlorate concentration provided as input and the concentration of the disinfectant and the chlorite concentration in the water. The calibration constant a computed can be stored in the memory internal or external to the processor 50 and can be used for all subsequent determination of the chlorate concentration until the next calibration measurement is done.

Referring still to FIG 2, once the calibration constant has been determined, the processor 50 is configured to determine the concentration of chlorate in the water using the function as expressed in equation (3). The measured concentration of the disinfectant in the water, the chlorite concentration, the calibration constant a and the exponential constant $x_1$, $x_2$ can be used in the function for determination of the chlorate concentration. According to an aspect, the chlorate concentration in the water can be determined for each measurement cycle of the sensors 40, 45, i.e., when the processor 50 is provided with the updated measured concentration of the disinfectant and the chlorite concentration.

A "processor" as used herein is a device for executing machine-readable instructions stored on a computer readable medium, for performing tasks and may comprise any one or combination of, hardware and firmware. For example, the processor may be implemented using a microcontroller, microprocessor, electronic devices, or other electronic units to perform the functions described herein or a combination thereof. The machine-readable instructions may be stored within the processor or external to the processor.

Referring still to FIG 2, according to an embodiment, the apparatus can further comprise one or more sensors to measure one or more additional parameters that influence the formation of chlorate in the water. The additional parameters, include, but not limited to, a temperature of the water, a pH value of the water and reaction time of the disinfectant with water process. For an example, according to an embodiment, the apparatus 35 can comprises a third sensor 60 adapted to measure the temperature of the water and output a third signal 52c responsive to the measured temperature. The third signal 52c is provided to the processor 50 via the analog to digital converter 55, depicted as 52c'. According to another embodiment, the apparatus can 35 comprises a fourth sensor 65 adapted to measure the pH value of the water and output a fourth signal 52d responsive to the measured pH value of the water. The fourth signal 52d is provided to the processor 50 via the analog to digital converter 55, depicted as 52d'.

Referring still to FIG 2, in an aspect, for measuring the reaction time of the disinfectant, the processor 50 can be configured to start a timer algorithm responsive an input from a user. The input from the user provides an indication of the moment at which the disinfectant is added into the water. In an alternative implementation, in case the processor 50 is coupled to a dosing pump adapted for dosing the disinfectant into the water, the indication of the moment at which the disinfectant is added into the water can be provided by the dosing pump to the processor 50. Thereafter, as the timer is initiated, the processor 50 can extract the reaction time of the disinfectant from the lapsed time indicated by the timer. In another aspect, the reaction time can be measured based on flow rate of the disinfectant and a distance the disinfectant travels. For example, one or more flow meters can be arranged into the pipe through which the disinfectant is added into the water and signals from the flow meters can be provided to the processor 50.

Referring still to FIG 2, the processor 50 can be configured to determine the chlorate concentration in the water using the function as expressed in equation (4). As one or more of the additional parameters can be included in the function, the apparatus can comprise the respective means for measuring the additional parameters. Thus, the function can include the additional parameters which are being measured. Similar to the exponential constants $x_1$, $x_2$, one or more of the respective exponential constants $x_3$, $x_4$, $x_5$, depending on the additional parameters the function includes, can be determined prior to the apparatus 35 being installed for determining the chlorate concentration in the water and can be stored in the memory.

Referring still to FIG 2, according to an embodiment, the function can further include the amount of consumption of the disinfectant. For example, in the shown example of FIG 2, the amount of consumption of the disinfectant can be determined by the processor 50 as the difference between the concentrations of the disinfectant measured by the first sensor 40 at two different time instances. The processor 50 can be configured to subtract the concentration of the disinfectant at a second time instance from the concentration of the disinfectant at a first time instance, the first time instance being the reference time instance and the second time instance being the later time instance. In an aspect, the processor 50 can be configured to compute the amount of consumption of the disinfectant continuously by subtracting the concentration of the disinfectant at the current time instance with the concentration of the disinfectant at the time instance the disinfectant was added to the water. In this case, the first time instance can be the time instance the disinfectant was added into the water and the current time instance can be the second time instance. According to an aspect, if the apparatus 35 is deployed for continuous measurement of the chlorate concentration, the processor 50 can be configured to determine the amount of consumption of the disinfectant in the water for each measurement cycle at which the first sensor 40 measures the concentration of the disinfectant in the water. The exponential constant $x_6$ can be determined prior to the apparatus 35 being installed for determining the chlorate concentration in the water and can be stored in the memory. The processor 50 can be configured to determine the chlorate concentration in the water concentration using the function as expressed in equation (5) or (6).

In the shown examples of FIG 2, it is shown that the concentration of the disinfectant and the chlorate concentration in the water are measured by the first sensor 40 and the second sensor 45 respectively. However, according to an embodiment, the concentration of the disinfectant in the water can be determined by knowing the amount of disinfectant added into the water. For example, if a dosing pump is used for dosing the disinfectant into the water, the amount of disinfectant being added into the water can be provided from the dosing pump to the processor 50. According to another embodiment, the apparatus 35 can comprise a sensor to measure the amount of disinfectant being added into the water and the processor 50 can be configured to receive the measured amount of disinfectant being added from the sensor. Accordingly, the processor 50 can be configured to determine the concentration of disinfectant in the water from the amount of disinfectant added into the water and from a time $t_d$ lapsed after the addition of the disinfectant. For example, as the disinfectant is decomposed on reaction with the water, the concentration of the disinfectant can be determined as a function of the time $t_d$. The function to determine the concentration of the disinfectant can be stored into a memory coupled to the processor 50 and the processor 50 can determine the concentration of the disinfectant by using the function. Thus, accordingly, the apparatus 35 may not comprise the first sensor 40 for measuring the concentration of the disinfectant in the water.

In the shown example of FIG 2, the chlorite concentration in the water is measured by the second sensor 45. However, according to an embodiment, the chlorite concentration in the water can be determined based on the calibration measurement. Thus, according to an embodiment, the calibration constant a computed can include the chlorite concentration in the water and can be used for all subsequent determination of the chlorate concentration in the water until the next calibration measurement is done. According to another embodiment, the chlorite concentration of the water can be determined based on a difference of the concentration of the disinfectant at two different time instances. The processor 50 can be configured to determine the chlorite concentration based on the difference of the concentration of the disinfectant determined at two different time instances. For example, the processor 50 can be configured determine the chlorite concentration using a relation of decomposition of the disinfectant into chlorite and chlorate. For example, the processor 50 can be configured to determine the chlorite concentration as a percentage of the amount of consumption of the disinfectant. The percentage can be predetermined and stored into a memory coupled to the processor 50. Thus, accordingly, in the aforementioned embodiments, the apparatus 35 may not comprise the second sensor 45 for measuring the chlorite concentration of the water.

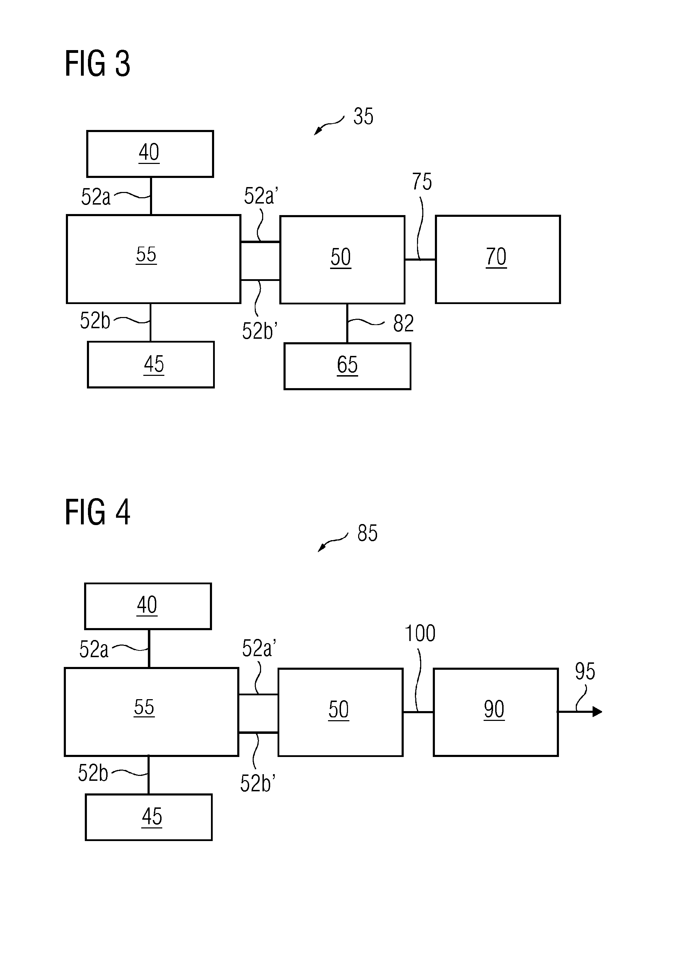

FIG 3 illustrates an exemplary block diagram of an apparatus 35 for measuring a concentration of chlorate in water according to a second embodiment. The apparatus 35 further comprises a display 70 operably coupled to the processor 50. The processor 50 on computing the concentration of chlorate in the water is configured to provide to the display 70 a concentration signal 75 indicative of the concentration of chlorate. The display 70 is configured to display the concentration of chlorate in the water responsive to the concentration signal 75 received from the processor 70. As the display 70 provides visual indication of the chlorate concentration, a user can know the chlorate concentration in the water by visually inspecting the display 70. The chlorate concentration computed can be displayed by the display 70 in a continuous manner and can be refreshed after each measurement cycle. In an aspect, the apparatus 35 can further comprise an audio device 80 operably connected to the processor 50. The processor 50 can be configured to provide an alarm signal 82 to the audio device 35 when the concentration of chlorate in the water is equal to or greater than a predetermined value. The predetermined value can be stored into a memory either internal or external to the processor 50 and can be selected such that the same provides an indication that the concentration of chlorite in the water is within the safe limits of human usage or consumption. For example, the predetermined value can be selected on the higher side of the safe limits of the concentration of chlorate prescribed by a regulation body. The audio device 80 can be configured to raise an alarm responsive to the alarm signal 82 received form the processor 50. A user responsive to the alarm can know that the concentration of chlorate in the water is high and take immediate measures to control the dosage of the disinfectant and dilute the concentration of chlorate by adding more un-disinfected water to the disinfected water.

FIG 4 illustrates an exemplary block diagram of a system 85 for disinfecting water with a chlorine dioxide based disinfectant according to an embodiment herein. In the shown example of FIG 4, the system 85 comprises a dosing pump 90 adapted to dose the disinfectant 95 into the water and the apparatus 35 of FIG 2. The dosing pump 90 is operably coupled to the processor 50. In an aspect, the processor 50 can be configured to generate a control signal 100 responsive to the concentration of chlorate computed. The control signal 100 is generated such that the concentration of chlorate in the water generated due to the addition of the disinfectant into the water is within the prescribed limits. The generated control signal 100 can be provided to the dosing pump 90 and the dosing pump 90, is controllable responsive to the control signal 100 for dosing of the disinfectant 95. Thus, the dosing of the disinfectant 95 can be controlled responsive to the concentration of chlorate in the water. This enables in efficient control of the concentration of chlorate in the water as the measurement of concentration of chlorate is performed in a continuous manner, and thus, enables in maintaining the concentration of chlorate within the prescribed limits.

Example

The following example illustrates determination of chlorate concentration in water using the embodiments described above. For the purpose of this example, following assumptions have been made as shown in table 1 below:

Table 1:

|  | Symbol | Values |
|---|---|---|
| Concentration of disinfectant in water | $C$ | 0.2 mg/L |
| Concentration of chlorite in water | $X$ | 0.13 mg/L |
| pH value of water | $pH$ | 7 |
| Temperature of water | $T$ | 25°C |
| Reaction time | $t$ | 10s |
| Exponential constant | $X_1$ | 1.27 |
| Exponential constant | $X_2$ | 0.45 |
| Exponential constant | $X_3$ | 0.9 |
| Exponential constant | $X_4$ | 0.27 |
| Exponential constant | $X_5$ | 0.20 |
| Calibration constant | $a$ | 0.1 |

In this example, the chlorate concentration in water is determined using equation (4).

$$\text{Chlorate (mg/L)} = a(C^{x_1})(X^{x_2})(pH^{x_3})(T^{x_4})(t^{x_5}) \tag{4}$$

Substituting the assumptions of table 1 in equation (4):

$$\text{Chlorate (mg/L)} = 0.1(0.2^{1\,27})(0.13^{0\,45})(7^{0\,9})(25^{0\,27})(10^{0\,2})$$

$$\text{Chlorate (mg/L)} = 0.1 \ (0.12951143)(0.3992772)(5.76219878)(2.38475519)$$
$$(1.58489319)$$

$$\text{Chlorate (mg/L)} = 0.11261977$$

The embodiments described herein can be used for measuring concentration of chlorate in water generated due to addition of a chlorine dioxide based disinfectant into water. Additionally, as the chlorate concentration in the water is determined without using the organic carbon content in the water directly, the chlorate concentration in the water can be determined in a continuous manner. Moreover, measuring the concentration of the chlorate in a continuous manner provides the advantage of more precise control as preventive measures can be initiated as soon as the concentration of chlorate in the water reaches or is near about the specified permissible limit. Advantageously, the determination of chlorate concentration can be more accurate for water having less than 10 mg/L of total organic carbon content, such as drinking water.
While this invention has been described in detail with reference to certain preferred embodiments, it should be appreciated that the present invention is not limited to those precise embodiments. Rather, in view of the present disclosure which describes the current best mode for practicing the invention, many modifications and variations would present themselves, to those of skill in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

**Claims**

1. A method for determining a chlorate concentration in a process of treating water with chlorine dioxide as a disinfectant, comprising:

   - determining a concentration of the disinfectant in the water,
   - determining a chlorite concentration in the water,
   - providing a function to determine the chlorate concentration depending on the concentration of the disinfectant and the chlorite concentration based on known chemical relations for the generation of chlorate and chlorite in the process of treating water with the disinfectant, and
   - applying the function to determine the chlorate concentration based on the concentration of disinfectant and the chlorite concentration.

2. The method according to claim 1, wherein the dependency of the function on the concentration of the disinfectant and/or the chlorite concentration is adjusted based on at least one calibration measurement.

3. The method according to claim 1 or 2, wherein the concentration of the disinfectant is determined by measuring the concentration of the disinfectant in the water.

4. The method according to any of the claims 1 to 3, wherein the function is based on the chemical relations in the form of a chemical relationship for the chlorite concentration depending on the concentration of the disinfectant and in form of a chemical relationship for the chlorate concentration depending on the concentration of the disinfectant.

5. The method according to claim 4, wherein the chemical relationships for the chlorite concentration and the chlorate concentration depend on the organic carbon content in the water and the function is established by eliminating the organic carbon content in the chemical relationships.

6. The method according to any of the claims 1 to 5, wherein the chlorite concentration is determined by a measurement of chlorite concentration in the water.

7. The method according to any of the claims 1 to 5, wherein the chlorite concentration is determined based on a calibration measurement of the chlorite concentration in the water, wherein the chlorite concentration is assumed as constant in the subsequent application of the function.

8. The method according to any of the claims 1 to 5, wherein the chlorite concentration is determined based on a difference of the concentration of the disinfectant which is measured at two different time instances.

9. The method according to any of the claims 1 to 8, wherein the function includes at least one additional parameter which influences the water treatment process.

10. The method according to claim 9, wherein the at least one additional parameter comprises at least one of temperature, pH value and reaction time of the disinfectant with the water.

11. The method according to claim 9 or 10, further comprising a step of measuring at least one value for the at least one additional parameter.

12. The method according to any of the claims 1 to 11, wherein the steps of:

   - determining the concentration of the disinfectant,
   - determining the chlorite concentration, and

- applying the function to determine the chlorate concentration

are repeated continuously.

13. The method according to claim 12 in reference to any of the claims 9 to 11, wherein the step of measuring the at least one value for the at least one additional parameter is repeated continuously.

14. An apparatus (35) determining a chlorate concentration in a process of treating water with chlorine dioxide as a disinfectant, comprising:

   - means for determining a concentration of the disinfectant in the water,
   - means for determining a chlorite concentration in water,
   - a processor (50) configured to determine the chlorate concentration depending on the concentration of the disinfectant and the chlorite concentration by applying a function based on known chemical relations of formation of chlorate and chlorite in the process of treating water with the disinfectant.

15. The apparatus (35) according to claim 14, wherein the means for determining a concentration of the disinfectant includes at least one first sensor (40) adapted to measure the concentration of the disinfectant in the water.

16. The apparatus (35) according to claim 14 or 15, wherein the means for determining a chlorite concentration includes at least one second sensor (45) adapted to measure the chlorite concentration in water.

17. The apparatus (35) according to any of the claims 14 to 16 adapted to perform the method according to any of the claims 1 to 13.

FIG 1

FIG 2

## FIG 3

## FIG 4

Europäisches
Patentamt

European
Patent Office

Office européen
des brevets

# EUROPEAN SEARCH REPORT

Application Number

EP 11 16 1097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | YOON-JIN LEE, HEA-TAE KIM, UN-GI LEE: "Formation of Chlorite and Chlorate from Chlorine Dioxide with Han River Water", KOREAN JOURNAL OF CHEMICAL ENGINEERING, vol. 21, no. 3, 1 May 2004 (2004-05-01), pages 647-653, XP000002658496, * the whole document * | 1-17 | INV. G01N33/18 |
| A | FR 2 819 312 A1 (ATOFINA [FR]) 12 July 2002 (2002-07-12) * claims 1-8 * | 1-17 | |
| A | GILBERT G ET AL: "Minimizing chlorite ion and chlorate ion in water treated with chlorine dioxide", AMERICAN WATER WORKS ASSOCIATION. JOURNAL, AMERICAN WATER WORKS ASSOCIATION, DENVER, CO, US, vol. 82, no. 4, 1 April 1990 (1990-04-01), pages 160-165, XP008107383, ISSN: 0003-150X * the whole document * | 1 | |
| A | TSUNG-FEI TANG, GILBERT GORDON: "Quantitative Determination of Chloride, Chlorite, and Chlorate Ions in a Mixture by Successive Potentiometric Titrations", ANALYTICAL CHEMISTRY, vol. 52, 1 January 1980 (1980-01-01), pages 1430-1433, XP000002658497, * the whole document * | 1 | |

TECHNICAL FIELDS SEARCHED (IPC)

G01N
C02F

-/--

The present search report has been drawn up for all claims

| Place of search | Date of completion of the search | Examiner |
|---|---|---|
| The Hague | 8 September 2011 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 11 16 1097

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | C.C. HONG, W.H. RAPSON: "Analyses of chlorine dioxide, chlorous acid, chlorite, chlorate, and chloride in composite mixtures", CANADIAN JOURNAL OF CHEMISTRY, vol. 46, 1 January 1968 (1968-01-01), pages 2061-2064, XP000002658498, * the whole document * ----- | 1 | |

TECHNICAL FIELDS
SEARCHED      (IPC)

The present search report has been drawn up for all claims

| Place of search | Date of completion of the search | Examiner |
|---|---|---|
| The Hague | 8 September 2011 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 16 1097

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2011

| Patent document cited in search report | Publication date | Patent family member(s) | Publication date |
|---|---|---|---|
| FR 2819312 A1 | 12-07-2002 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEE.** Formation of chlorite and chlorate from chlorine dioxide with Han river water. *Korean J. Chem. Eng.,* vol. 21 (3